# EUROPEAN PATENT APPLICATION

(11) **EP 2 829 241 A1**
(43) Date of publication of application: **28.01.2015**
(21) Application number: 13177444.0
(22) Date of filing: 22.07.2013
(51) Int. Cl.: A61B 17/30, A61B 17/04

(54) **Fascia device and method to assist in suturing fascia of a person during surgery**

(71) Applicant: De Jong, Dr., Jeffrey, 1788 WT Julianadorp (NL)
(72) Inventor: De Jong, Dr., Jeffrey, 1788 WT Julianadorp (NL)
(74) Representative: De Vries & Metman

(57) **Abstract**

The present invention relates to a fascia device (1) for assisting in suturing fascia (7) of a person during surgery. The device (1) comprises a member (2, 3) adapted to be inserted in a surgical wound in the fascia (7) for protecting underlying tissue, such as an intestinal (8). The member (2, 3) has opposite first and second sides (9, 10), the first side (9) facing the underlying tissue (8) during use. The fascia device (1) is provided with an indentation (4) in the second side (10) of the member (2, 3) for looking under the fascia (7) of the person undergoing surgery.

## Description

The present invention relates to a fascia device for assisting in suturing fascia of a person during surgery.

The present invention also relates to a method to assist in suturing fascia of a person during surgery by means of a fascia device according to the invention.

During a surgery with an endoscope, such as a laparoscopy, a surgeon has to make at least one incision in the skin of a person undergoing the surgery in order to be able to enter the interior of the person, for example the abdominal cavity. When the surgery is almost over, the incision needs to be closed. The incision is closed in two steps in the case of a laparoscopy. The abdominal fascia, meaning the fascia that forms part of the general layer lining the walls of the abdominal cavity and investing the abdominal organs, is sutured first and thereafter the top layer of skin is sutured.

The suturing of the abdominal fascia is a risky part of the procedure, since it is possible that during suturing underlying tissue, such as an intestinal wall, is damaged by a suture needle. It is even possible that the underlying tissue gets attached to the abdominal fascia by means of the suture thread used during suturing.

The present invention has for its object to obviate or at least reduce such a drawback of the known art.

The invention provides for this purpose a fascia device for assisting in suturing fascia of a person during surgery, the device comprising:
a member adapted to be inserted in a surgical wound in the fascia for protecting underlying tissue, such as an intestinal, said member having opposite first and second sides, said first side facing the underlying tissue during use,
wherein an indentation is provided in the second side of the member for looking under the fascia of the person undergoing surgery.

The fascia device will be used during surgery at the moment that the surgeon wishes to close the incision because the main part of the procedure is completed. The fascia device according to the invention may be used in combination with a saddle hook. The saddle hook may be used to pull away a layer of skin and fat from the incision at a first position, such that the surgeon gets a improved overview of the incision. Subsequently, the fascia device is used the push down a layer of skin and fat, at a position opposite to the position where the saddle hook is applied, to provide access for the suture needle to the fascia of the person undergoing surgery. During pushing down the layer of skin and fat with the fascia device, the fascia device is at least partly, preferably about half of the indentation, placed under the fascia. When the fascia device is slightly tipped over, the surgeon is able to see by means of the indentation of the device whether tissue, such as an intestinal wall, underlying the fascia is separated from the fascia and not within the space between the device and the fascia. The surgeon may subsequently suture the fascia when tissue underlying the fascia is separate from the fascia.

A first advantage of the fascia device is that the surgeon may see whether the fascia is free and no underlying tissue is pinched between the device and the fascia. It is, therefore prevented that the surgeon starts suturing the fascia and thereby damages the underlying tissue such as an intestinal wall.

A further advantage is that the tissue underlying the member of the device is protected by the member, such that the suture needle will hit the member, when the suture needle is pushed too deep for example, instead of damaging the tissue underlying the member.

The invention has various embodiments which will become apparent from the following description of several such embodiments. The advantageous inventive features of the invention in all its aspects, including the measures defined in de dependent claims, are by no means limited to the considerations stated above and/or below.

A further embodiment of the fascia device according to the invention the device comprises first and second portions situated opposite each other, wherein the first part forming the first side of the member and the indentation is formed by an aperture in said second portion forming the second side of the member. An advantage of this embodiment is that the space between the first and second portion may form a draining channel provided under the indentation and therefore any fluid is able to flow away very easily without any fluid remaining in the indentation. The user of the fascia device thereby keeps a clear view on the fascia device and thus the indentation.

A feature of another embodiment of the fascia device according to the invention is that the first and second portions of the member, at least during use, are in proximity to each other. As the parallel first and second portions are positioned at a small distance from each other, the indentation is completely open on the sides so that any blood there will flow away and it is not possible that blood will remain in the indentation, which could hinder view on the fascia.

In a further embodiment of the fascia device according to the invention the aperture is also adapted for guiding a needle, in particular a suture needle. The aperture is used to see if no tissue is present between the fascia device and the fascia to be sutured and the surgeon may accordingly determine that it is safe to suture the fascia. When the surgeon has determined that there is no tissue between the fascia device and the fascia, it is evident that a space for safely suturing is available. By guiding the suture needle through the aperture of the second member during suturing of the fascia, it is prevented that tissue besides the fascia device gets damaged by the suture needle.

It is, further, advantageous that at least a part of the first portion of the member situated opposite the aperture in the second portion is adapted for guiding the needle. Due to the suture needle being guided by a part the first portion of the member, it is possible to control how deep the suture needle is inserted into the tissue. When the needle is inserted into the tissue too deep, the underlying tissue can be damaged. This is prevented by guiding the needle during suturing, since at least the depth of entering the needle is controlled.

The first and second portions of the member may be interconnected at one end. An advantage of this embodiment is that a positional configuration of the first portion and the second portion remains the same during use of the fascia device.

In a further embodiment of the fascia device according to the invention, one end of the member, which end is to be inserted first during use, has a rounded shape. Surgery has as an object to treat the person undergoing surgery. By providing the end to be inserted with a rounded shape, it is prevented that damage is caused by the fascia device to the tissue at the location of where the fascia device is applied.

It is advantageous that the member is elongated. The fascia device is adapted and intended for, inter alia, pushing down skin and/or fat during use of the fascia device. By providing an elongated member it is effectuated that the force applied to the fascia device and thus indirectly the skin of the person undergoing surgery is extended over a bigger area. It is thus prevented that damage is caused by the fascia device to the skin, or at least the amount of damage caused to the tissue is reduced.

Another embodiment of the fascia device according to the invention is **characterized in that** the member is provided with at least a holding section on the end remote from said indentation. It is, thereby, advantageous that each of the first and second portions of the member is provided with a holding section. It is effectuated that a user of the fascia device has the possibility to hold the fascia device firmly during use thereof.

It is, therefore, advantageous, that the fascia device is produced from sterilizable material. The costs of surgical tools are often relative high due to the demands, which are often statutory, regarding, e.g., performance and/or material properties. It is conceivable that a user of the fascia device does not wish to cause any unintended damage to the person undergoing surgery, therefore the demands apply for the fascia device and the like. When the fascia device is produced from sterilizable material, it is possible to sterilize the fascia device and to use it again during another procedure.

A feature of a preferred embodiment of the fascia device is that the fascia device is incorporated into or attached to a medical device. It is in general policy that during a surgery as little as possible instruments are present in a working area of the surgeon. An object of this policy is preventing that instruments get lost during surgery or even worse, remain in the patient after surgery. By incorporating the fascia device into or attaching it to a medical device, it is prevented that an additional instrument is introduced into the working area of the surgery. It is thus accomplished to provide the surgeon with the fascia device without introducing an additional separate instrument.

It is advantageous that the fascia device is incorporated in a pair of tweezers having two legs interconnected at one end, the indentation being provided near said one end and the legs forming holding sections. It is common that almost every surgery is terminated with suturing the surgical wound of the patient. Furthermore, a pair of tweezers is used during almost every surgical procedure, such as a laparoscopy. Is it therefore advantageous when the fascia device has the form of a pair of tweezers, since the pair of tweezers in this manner has two functions and the surgeon has the option to use the fascia device, but no additional physical instruments are introduced into the working area of the surgeon.

The invention further relates to a method of assisting in suturing fascia of a person during surgery by means of a fascia device according to any one of the preceding claims, the method comprising steps of:
- pulling a top layer of skin at an edge of a surgical wound in a direction away from said surgical wound for providing access to the underlying fascia;
- inserting said fascia device at least partly into the surgical wound for pushing down a layer of skin situated opposite the fascia freed by pulling away the top layer of skin;
- tipping over the fascia device to see whether tissue lying under the fascia is separated therefrom; and
- suturing the fascia of the person.

The method has at least some of the advantages which are mentioned in relation to the fascia device according to the invention. An important advantage is that a surgeon may check before suturing whether the fascia is free of underlying tissue and therefore it may be prevented that damage is caused to the underlying tissue during suturing of the fascia.

The invention further relates to a pair of tweezers comprising a fascia device according to any one of the preceding claims. The pair of tweezers has at least some of the advantages mentioned above in relation to the fascia device.

Following below is a description of an embodiment of the fascia device as shown in the accompanying drawings and provided only by way of example, and in which:
Fig. 1 is a perspective view of the embodiment of a fascia device according to the invention;
Fig. 2 is a plan view of a part of the fascia device of Fig. 1.
Fig. 3 is an enlarged sectional view according to the line III-III in Fig. 2.
Fig. 4 is a schematic illustration of how the fascia device is positioned with respect to the fascia during suturing;

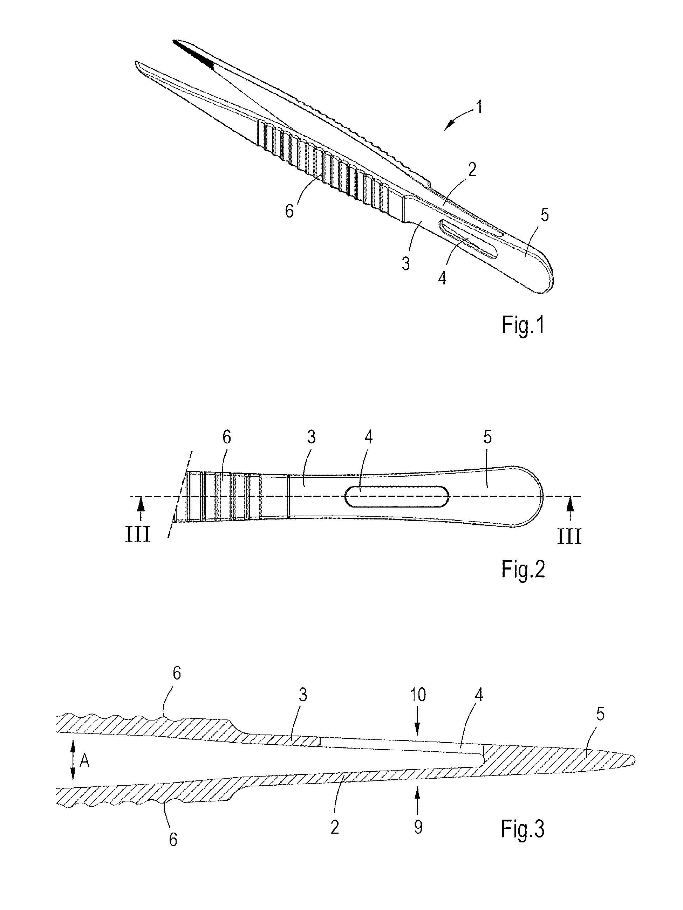

Fig. 1 - 3 show a pair of tweezers provided with a fascia device 1 according to the invention at one end of the pair of tweezers. As can be seen in the figure, the fascia device 1 comprises a member, which member comprises a first portion 2 and a second portion 3 situated opposite each other. The first portion 2 forms the first side 9 of the member and the second portion 3 forms the second side 10 of the member.

In this embodiment the first portion 2 and the second portion 3 are elongated and are formed by a part of legs of the pair of tweezers, wherein one of the legs of the pair of tweezers is provided with an indentation, here in the form of an aperture 4, which is adapted to look through towards and past the first portion 2. The second portion 3 of the fascia device 1 is situated opposite the first portion 2 of the device 1. It is clear from Fig. 1 that is possible to look through the aperture 4 provided in the second portion 3 towards and past the first portion 2. At least one of the first portion 2 and the second portion 3 is provided with a holding section 6. As is apparent from Fig. 1 - 3, the holding sections 6 are formed by ridges provided on the legs of the pair of tweezers. The user of the fascia device 1 is able to hold the device firmly due to the holding section(s) 6, which is advantageous for, e.g., pushing down the tissue in order to provide access to the fascia for the suture needle. The holding sections 6 are provided on the remote end from the aperture 4.

The two legs of the pair of tweezers are interconnected at one end 5, the aperture 4 being provided near the one end 5 and the legs forming holding sections 6. In this embodiment, the member comprising the first portion 2 and the second portion 3 is incorporated into the legs of the pair of tweezers between the holding sections 6 and the one end 5.

As can be seen in Fig. 3, at least a part 11 of the first portion 2 of the member situated opposite the aperture 4 is adapted for guiding a needle. As mentioned above, the indentation is here in the form of the aperture 4, and part 11 of the first portion 2 forms the bottom of the indentation. As the parallel first and second portions 2, 3 are positioned at a small distance from each other (also if the legs of the pair of tweezers are pinched together), the "indentation" is completely open on the sides so that any blood there will flow away and it is not possible that blood will remain in the "indentation", which could hinder view on the fascia.

The end 5 where the first portion 2 and the second portion 3 are interconnected has a rounded shape, as shown in Fig. 1 - 3. Since the end 5 is used to push down tissue surrounding an incision made during surgery, it is desirable to prevent causing any additional damage to the tissue by pushing down thereof. The rounded shape of the end 5 has the advantage that no damaged is caused to the tissue by the end 5. During use of the fascia device 1, the first portion 2 and the second portion 3 are moved towards each other in direction A, such that the portions 2, 3 are substantially lying against each other.

It is noticed that the dimensions of the fascia device are slightly smaller than the dimensions of the surgical incision to be sutured. It is, therefore, prevented that damage is caused, e.g. the incision becomes larger due to insertion of the fascia device, to the tissue surrounding the surgical incision, for example by tearing tissue surrounding the surgical incision.

The dimensions of the aperture 4 are dependent on or may be adjusted to the intended use of the fascia device. It is conceivable that, when a smaller incision is needed for surgery, the fascia device and thus the aperture provide in the second member thereof may be smaller. For example, a longitudinal dimension of the aperture in the case that the fascia device is used during a laparoscopy may be in a range between 10-25 mm, preferably 11-17 mm, or even more preferred 12-15 mm.

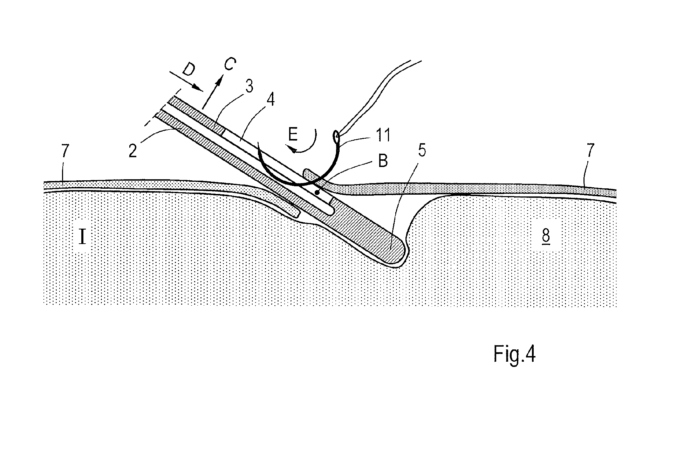

Fig. 4 shows schematically how the fascia device is positioned with respect to the fascia during use. The fascia device 1 has pushed down the fascia 7. The top layer of skin is not shown in the figures in order to provide a better overview, which top layer of skin is pulled away in order to provide access to the fascia 7. It is remarked that the fascia device 1 of Fig. 4 may be the fascia device of Fig. 1 in squeezed state, however it is also possible that the fascia device is of another shape.

As can be seen in Fig. 4, the first side 9 of the fascia device 1 faces the fascia 7 pushed down by the fascia device 1. When the device 1 is in the position as shown in Fig. 4, the user may tip over the fascia device 1 in the direction of arrow C thereby rotating around axis B. Subsequently, the user is able to look in the direction of arrow D under the fascia 7 to determine whether the fascia 7 is contact with any underlying tissue I, such as an intestinal wall 8.

When the user of the fascia device sees that the fascia is separated from any underlying tissue, such as an intestinal wall 8, then the user may start suturing of the incision. In general, suturing is performed in one direction as indicated by arrow E. The depth the suture needle is allowed to be inserted is also controlled by the fascia device 1. Since the part of the fascia to be sutured is lying above the fascia device 1, the underlying tissue I is protected by the fascia device 1. Further, the suture needle is guided by the aperture 4 during suturing.

As shown in Fig. 4, the suture needle 11 only penetrates the fascia 7 on one side of the incision. When the suture needle 11 has penetrated the fascia on one side of the incision, the fascia device 1 is pulled out of the incision and is subsequently inserted again in opposite direction. As a result, the fascia 7 on the other side of the incision is on top of the fascia device 1. The surgeon now may slightly tip over the fascia device 1 in order to see if underlying tissue I is separated from the fascia and, if so, go on with suturing the fascia 7.

After examination of the foregoing many alternative and additional embodiments will occur to the skilled person, all lying within the scope of the present invention as defined in the appended claims. For example, it is conceivable to provide the member of the fascia device with a single portion having an indentation in the second side of the single portion. It is, further, conceivable to provide at least one draining channel in fluid connection with the indentation, in order to drain any fluid from the indentation.

## Claims

1. Fascia device for assisting in suturing fascia of a person during surgery, said device comprising:
- a member adapted to be inserted in a surgical wound in the fascia for protecting underlying tissue, such as an intestinal, said member having opposite first and second sides, said first side facing the underlying tissue during use
wherein an indentation is provided in the second side of the member for looking under the fascia of the person undergoing surgery.

2. Fascia device according to claim 1, wherein said member comprises first and second portions situated opposite each other, wherein the first portion forming the first side of the member and the indentation is formed by an aperture in said second portion forming the second side of the member.

3. Fascia device according to claim 2, wherein said first and second portions of the member, at least during use, are in proximity to each other.

4. Fascia device according any one of the preceding claims, wherein said indentation is also adapted for guiding of a needle, in particular a suture needle.

5. Fascia device according to any one of the claims 3 to 5, wherein at least a part of said first portion of the member situated opposite said aperture in the second portion is adapted for guiding said needle.

6. Fascia device according to any one of the claims 3 to 6, wherein said first and second portions of the member are interconnected at one end.

7. Fascia device according to any one of the preceding claims, wherein one end of said member, which end is to be inserted first during use, has a rounded shape.

8. Fascia device according to any one of the preceding claims, wherein said member is elongated.

9. Fascia device according to any one of the preceding claims, wherein the member is provided with at least a holding section on the end remote from said indentation.

10. Fascia device according to any one of the preceding claims, wherein each of said first and second portions comprises a holding section.

11. Fascia device according to any one of the preceding claims, wherein said fascia device is produced from sterilizable material.

12. Fascia device according to any one of the preceding claims, wherein said fascia device is incorporated into or attached to a medical device.

13. Fascia device according to any one of the preceding claims, wherein said fascia device has the form of a pair of tweezers having two legs interconnected at one end, the indentation being provided near said one end and the legs forming holding sections.

14. Method of assisting in suturing fascia of a person during surgery by means of a fascia device according to any one of the preceding claims, the method comprising steps of:
- pulling a top layer of skin at an edge of a surgical wound in a direction away from said surgical wound for providing access to the underlying fascia;
- inserting said fascia device at least partly into the surgical wound for pushing down a layer of skin situated opposite the fascia freed by pulling away the top layer of skin;
- tipping over the fascia device to see whether tissue lying under the fascia is separated therefrom; and
- suturing the fascia of the person, preferably in two opposite directions.

15. Pair of tweezers comprising a fascia device according to any one of claims 1 - 13.
